# EUROPÄISCHE PATENTSCHRIFT

(11) **EP 1 353 654 B1**
(45) Veröffentlichungstag und Bekanntmachung des Hinweises auf die Patenterteilung: **08.06.2005**
(21) Anmeldenummer: 02703547.6
(22) Anmeldetag: 14.01.2002
(51) Int. Cl.: A61K 9/70

(54) **VORRICHTUNGEN UND VERFAHREN ZUR HITZEPULS-GESTÜTZTEN TRANSDERMALEN APPLIKATION VON WIRKSTOFFEN**
DEVICES AND METHODS FOR HEAT-PULSE ASSISTED THERMAL APPLICATIONS OF ACTIVE SUBSTANCES
DISPOSITIFS ET PROCEDES PERMETTANT L'APPLICATION TRANSDERMIQUE ASSISTEE PAR IMPULSIONS THERMIQUE, DE SUBSTANCES ACTIVES

(30) Priorität: 23.01.2001 DE 10102817
(43) Veröffentlichungstag der Anmeldung: 22.10.2003
(73) Patentinhaber: LTS Lohmann Therapie-Systeme AG, 56626 Andernach (DE)
(72) Erfinder: HORSTMANN, Michael, 56564 Neuwied (DE); BRACHT, Stefan, 07751 Cospeda (DE)
(74) Vertreter: Flaccus, Rolf-Dieter, Dr.
(86) Internationale Anmeldenummer: PCT/EP2002/000286
(87) Internationale Veröffentlichungsnummer: WO 2002/058678

(56) Entgegenhaltungen:
- WO-A-00/18338
- WO-A-01/64151
- WO-A-85/02124
- DE-A- 2 931 610
- US-A- 4 398 535

## Beschreibung

Die Erfindung betrifft Vorrichtungen zur transdermalen Applikation von Wirkstoffen, wobei diese Vorrichtungen eine lokale Erwärmung der Applikationsstelle zum Zwecke der Permeationsverbesserung bewirken. Die Erfindung betrifft insbesondere Vorrichtungen der genannten Art, welche ein wirkstoffhaltiges transdermales therapeutisches System (TTS) enthalten, sowie wirkstofffreie Vorrichtungen der genannten Art zur Verabreichung von Hitzepulsen an die menschliche oder tierische Haut.
Die Erfindung umfaßt des weiteren Verfahren zur transdermalen Verabreichung von Wirkstoffen, bei welchen die Wirkstoffpermeation durch Temperaturerhöhung gesteigert wird.

Transdermale therapeutische Systeme (TTS) sind Vorrichtungen oder Darreichungsformen, die einen oder mehrere Arzneistoffe in vorausbestimmter Rate kontinuierlich über einen festgelegten Zeitraum an einem festgelegten Anwendungsort, d. h. ein bestimmtes Hautareal, abgeben. Gegenüber oralen Darreichungsformen sind TTS deshalb vorteilhaft, weil sie die Verabreichung von systemisch wirkenden Stoffen unter Umgehung des Magen-Darm-Traktes ermöglichen, wodurch die sonst infolge des "first-pass"-Effekts rasch einsetzende Metabolisierung und Inaktivierung vermieden bzw. hinausgezögert wird. Hieraus resultiert eine verbesserte Wirkstoffausnutzung. Zudem lassen sich auf diese Weise gewisse Nebenwirkungen vermeiden, die bei oraler Verabreichung häufig auftreten können. Von Vorteil ist außerdem, daß diese Systeme eine kontinuierliche und konstante Zufuhr von Wirkstoffen ermöglichen, so daß der Blutplasmaspiegel auf einem weitgehend konstanten Niveau gehalten wird.

Die Therapie verschiedener Erkrankungen mit systemischen Medikamenten, die über die Haut in den Körper gelangen, ist mittlerweile weithin bekannt und beschrieben. Eine Reihe von Arzneistoffen, z. B. Nikotin, Nitroglycerin, Clonidin, Estrogen und Gestagene, sind bereits in Form von transdermalen therapeutischen Systemen im Handel.

Der Aufbau eines TTS umfaßt typischerweise die folgenden Bestandteile:
- ein wirkstoffhaltiges Reservoir;
- eine mit diesem Reservoir verbundene Rückschicht auf der hautabgewandten Seite;
- eine haftklebende Schicht zur Befestigung des Systems auf der Haut (diese kann auch entfallen, wenn das wirkstoffhaltige Reservoir selbst haftklebend ist);
- eine ablösbare Schutzschicht, welche die haftklebende Hautkontaktseite bedeckt und vor der Applikation abgelöst wird.

Das wirkstoffhaltige Reservoir kann beutelförmig sein oder eine feste Matrix darstellen.
Im Falle eines beutelförmigen Reservoirs ist die Rückseite des flachen Beutels für den Wirkstoff undurchlässig und die Kontaktseite des Beutels zur Haut wird aus einer durchlässigen Membran gebildet wird. Der Wirkstoff befindet sich in einem solchen System in Form einer flüssigen oder halbfesten Zubereitung im Innern des Beutels.
Bei TTS vom "Matrix"-Typ befindet sich der Wirkstoff als Lösung oder Dispersion innerhalb einer geeigneten Polymermatrix, welche als Wirkstoffreservoir dient. Als haftklebende Grundpolymere für die Matrixschicht(en) werden beispielsweise Acrylatpolymere oder Acrylatcopolymere, Polyisobutylene, Styrol-Isopren-Blockcopolymere oder Polysilikone verwendet. Falls erforderlich, kann die wirkstoffhaltige Matrix auch aus mehreren Schichten aufgebaut sein.

Bei den vorstehend beschriebenen TTS erfolgt die Wirkstoffabgabe an die Haut grundsätzlich auf dem Wege der passiven Diffusion. Ein großes Problem bei der Zufuhr pharmazeutischer Wirkstoffe über die Haut besteht in der Begrenztheit der menschlichen Haut, solche Stoffe mit ausreichender Dosierungsgeschwindigkeit aufzunehmen. Dies ist auch der Hauptgrund dafür, daß zur Zeit nur eine relative geringe Anzahl von Wirkstoffen für die transdermale Therapie geeignet ist.
Andererseits sind Möglichkeiten bekannt, um die Permeation von Wirkstoffen durch die Haut zu steigern. Dies kann beispielsweise durch Verwendung von permeationsverstärkenden Substanzen erreicht werden, oder durch eine Erhöhung der thermodynamischen Aktivität des Wirkstoffs im Wirkstoffreservoir. Jedoch reichen bei vielen Wirkstoffen auch diese Maßnahmen nicht aus, weshalb es weiterer Steigerungsmöglichkeiten bedarf.
Die Verwendung von Zusatzstoffen, wie permeationsverbessernden Stoffen, hat zudem den Nachteil, daß es dabei zu Unverträglichkeiten auf der Haut kommen kann.
Es besteht daher der Wunsch, die Hautpermeation weiter zu steigern oder um ein Mehrfaches zu erhöhen, ohne daß dabei die Haut geschädigt wird oder Mißempfindungen auftreten, wie dies bei der Verwendung von permeationsverbessernden Substanzen häufig der Fall ist.

Es ist grundsätzlich bekannt, daß die Permeationsgeschwindigkeit von Wirkstoffen durch die Haut bei erhöhter Temperatur schneller verläuft. Jedoch gibt es nur geringe Möglichkeiten, diesen Effekt auch praktisch auszunutzen, da die normale Hauttemperatur von etwa 32 °C nur unwesentlich gesteigert werden kann, ohne daß Mißempfindungen auftreten. Infolgedessen wurde von dieser Möglichkeit bislang wenig Gebrauch gemacht.

Die Schmerzwahrnehmung auf der Haut setzt gewöhnlich ein, wenn die Hauttemperatur über einen Wert von ca. 45 °C erhöht wird. Dadurch sind die Möglichkeiten der Ausnutzung eines Temperatureffekts zur Permeationsverbesserung stark eingeschränkt. So konnte die in mehreren wissenschaftlichen Publikationen bestätigte Permeationssteigerung durch erhöhte Temperatur (z. B. Watanabe Y, Hongo S, Matsumoto M: "Evaluation of excised loach skin for studies on transdermal permeation of drugs in vitro"; Yakugaku Zasshi, 1989 Sep., 109:9, 656-661) in der therapeutischen Praxis bisher nicht realisiert werden.

WO 01 64151 A beschreibt eine Vorrichtung zur transdermalen Wirkstoffabgabe, wobei die Wirkstoffabgabe durch eine kontrollierte Wärmezufuhr gesteuert wird. Die Vorrichtung umfaßt ein Wirkstoffreservoir sowie ein elektrisches Heizelement, das in der Nähe des Wirkstoffreservoirs angeordnet ist. Alternativ kann eine chemische Stoffmischung zur Wärmeerzeugung verwendet werden.
Das elektrische Heizelement ist mit einer Steuereinheit verbunden, welche die Menge und Dauer der Wärmeerzeugung steuert. Die Wärmeerzeugung beginnt nach Betätigung eines Startknopfes und endet nach Ablauf einer vorgegebenen Zeitdauer.

WO 00 18338 A offenbart eine Vorrichtung zum transdermalen Verabreichen von Analgetika unter Anwendung von Wärme. Die Erwärmung wird vorzugsweise mit Hilfe einer exotherm reagierenden chemischen Stoffmischung bewirkt. Alternativ kann die Vorrichtung mit einem elektrischen Heizelement und einer damit verbundenen Steuereinheit ausgestattet sein, wodurch eine gesteuerte Erwärmung ermöglicht und die Dauer des Aufheizvorgangs begrenzt wird.

Die Verwendung von künstlich erzeugter Wärme auf der Haut ist auch deshalb problematisch, weil die üblichen Wärmeanwendungsgeräte in der Regel einen hohen Stromverbrauch aufweisen und deshalb nicht in tragbarer Form zur Verfügung stehen. Gleichwohl sind verschiedene elektrische Geräte beschrieben worden, mit welchen die Haut für eine gewisse Zeit erwärmt oder abgekühlt werden kann. In der Regel werden diese Geräte für physikalisch-therapeutische Zwecke an Patienten eingesetzt (vgl. US 5 746 702 und US 5 097 828).

Es war deshalb die Aufgabe der vorliegenden Erfindung, eine Vorrichtung bereitzustellen, welche die transdermale Verabreichung von Wirkstoffen ermöglicht und welche eine Steigerung der Wirkstoffpermeation ermöglicht, die nicht auf der Anwesenheit von permeationsfördernden Zusatzstoffen beruht, und die eine schmerzende Schädigung oder Reizung der Haut vermeidet.

Die Lösung dieser Aufgabe gelingt überraschenderweise mit einer Vorrichtung gemäß Anspruch 1 oder Anspruch 2, bzw. mit einem Verfahren gemäß Anspruch 14 oder 15, wobei die Unteransprüche weitere besonders nützliche Ausführungsformen der Erfindung betreffen.

Gemäß Anspruch 1 umfaßt die erfindungsgemäße Vorrichtung zur hitzepuls-gestützten transdermalen Verabreichung von Wirkstoffen ein transdermales therapeutisches System, das auf der hautzugewandten Seite ein wirkstoffdurchlässiges elektrisches Heizelement, oder mehrere solche Elemente aufweist. Außerdem umfaßt die erfindungsgemäße Vorrichtung eine mit diesem Heizelement verbundene Steuereinheit und eine Stromquelle, wobei die genannte Steuereinheit ein puls-artiges Aufheizen des genannten Heizelements bewirkt.

Das transdermale therapeutische System (TTS), welches Bestandteil der Vorrichtung nach Anspruch 1 ist, enthält neben dem bzw. den Heizelement(en) ein Wirkstoffreservoir und eine Rückschicht, welche das System auf der Außenseite bedeckt. Das Wirkstoffreservoir ist mit dem/den Heizelement(en) verbunden und liegt über dem Heizelement. Das Heizelement bzw. die Heizelemente steht/stehen während der Applikation in direktem Kontakt mit der Haut.

Die vorliegende Erfindung umfaßt ferner eine Vorrichtung zur Verabreichung von Hitzepulsen an die Haut gemäß Anspruch 2. Diese umfaßt ein haftklebendes medizinisches Pflaster zur Befestigung der Vorrichtung auf der Haut, ein oder mehrere elektrische Heizelemente, die mit diesem Pflaster verbunden sind, sowie eine mit diesem/n Heizelement(en) verbundene Steuereinheit und eine Stromquelle. Mittels der genannten Steuereinheit kann ein puls-artiges Aufheizen des genannten Heizelements bewirkt werden. Diese Vorrichtung gemäß Anspruch 2 enthält keinen Wirkstoff bzw. keine wirkstoffhaltige Schicht; sie kann als wirkstofffreies medizinisches Hitzepuls-Pflaster bezeichnet werden. Ihre Verwendung bei der Hitzepuls-gestützten Wirkstoff-Verabreichung erfolgt in der Weise, daß zunächst eine wirkstoffhaltige Zubereitung auf ein Hautareal appliziert wird und anschließend, gegebenenfalls nach einer Einwirkungszeit, die vorrichtung nach Anspruch 2 auf diese Hautfläche aufgebracht wird, wobei das/die Heizelemente in Kontakt mit der Hautoberfläche stehen.

Die nachfolgend beschriebenen Ausführungsvarianten beziehen sich grundsätzlich sowohl auf die vorrichtung nach Anspruch 1 als auch auf die Vorrichtung nach Anspruch 2. Allerdings wird darauf hingewiesen, daß die Heizelemente im Falle der Vorrichtungen nach Anspruch 1 wirkstoffdurchlässige Eigenschaften aufweisen müssen; im Falle der Vorrichtungen nach Anspruch 2 dies nicht erforderlich.

Das/die Heizelement(e) der erfindungsgemäßen Vorrichtungen ist/sind mit einer Stromquelle als energieversorgende Einrichtung gekoppelt; dabei wird mittels einer dazwischengeschalteten Steuereinheit bewirkt, daß in dem Heizelement bzw. den Heizelementen impulsweise Wärme erzeugt und freigesetzt wird, d. h. es wird ein puls-artiges Aufheizen der Heizelemente und der mit ihnen in Kontakt stehenden Hautoberfläche bewirkt.

Die impulsweise Zufuhr von Wärme an die Haut hat den Vorteil, daß bei entsprechender Oberflächen-Nahpositionierung eine hohe Wärmebeeinflussung der äußeren Hornschicht (Stratum corneum) erreicht wird, ohne daß tiefergelegene Hautschichten, die besonders hitzeempfindlich sind, geschädigt werden. Aufgrund der lokal sehr schnell freigesetzten Wärmemenge entsteht ein Wärmepuls, welcher die äußere Schicht des Stratum corneum wie auch die wirkstoffführende Schicht, das wirkstoffhaltige Reservoir, des TTS kurzzeitig erwärmt, vorzugsweise auf über 50 °C. Nach wenigen Sekunden wird die Wärme durch Wärmediffusion in die tieferen Hautschichten abgeleitet und erzeugt dort wegen des Wärmeausgleichs nur eine als nicht schmerzhaft wahrgenommene leichte Wärmeempfindung.

Die Aufheizung des Heizelements während der impulsweisen Wärmezufuhr liegt im Temperaturbereich zwischen 40 °C und 200 °C, bevorzugt im Bereich zwischen 50 °C und 120 °C. Die angegebenen Temperaturen (Zieltemperaturen) beziehen sich auf das Heizelement. Aufgrund des direkten Kontakts des Heizelements zur Haut wird davon ausgegangen, daß die genannten Temperaturen kurzzeitig auch in der oberen Hornschicht der Haut erreicht werden.

Bei einer bevorzugten Ausgestaltung der Erfindung ist vorgesehen, daß zur Erzeugung der Wärmepulse jeweils eine Stromstärke von 1 bis 100 A bei einer anliegenden Spannung von 1,2 bis 24 V über eine Pulsdauer von 0,01 bis 0,5 s angewendet wird. Besonders bevorzugt sind Stromstärken im Bereich von 5 bis 20 A bei Spannungen im Bereich von 1,2 bis 7,2 V über eine Pulsdauer von 0,05 bis 0,1 s.

Die Dauer der Wärmepulse wird begrenzt und beträgt vorzugsweise 0,1 bis 2 Sekunden, besonders bevorzugt 0,2 bis 1 s. Die Temperatur oder/und die Dauer der Wärmepulse durch die Steuereinheit vorgegeben oder ist durch diese steuerbar. Bei der Verabreichung von Wirkstoffen unter Verwendung der erfindungsgemäßen Vorrichtung werden im allgemeinen mehrere aufeinanderfolgende Wärmeimpulse abgegeben. Zwischen den einzelnen Wärmepulsen liegen jeweils Zeitintervalle, in denen die Ableitung der erzeugten lokalen Wärme in die tiefer gelegenen Hautschichten stattfinden kann. Diese Zeitintervalle dauern mindestens 0,01 Sekunden und können bis zu mehreren Stunden dauern, z. B. bis zu 10 h. Bevorzugt dauern die zwischen den einzelnen Pulsen liegende Zeitintervalle 0,1 bis 100 s, insbesondere 1 bis 60 s.

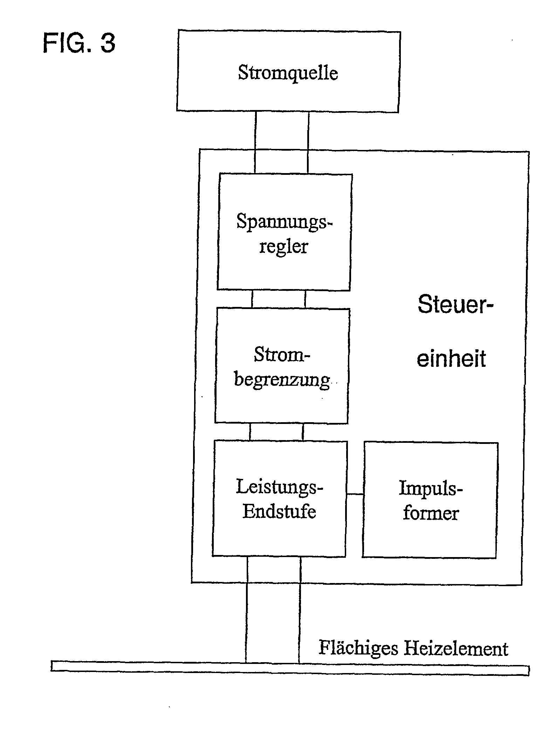

Die einzelnen Parameter, welche die Wärmepulse kennzeichnen, können mit Hilfe der genannten Steuereinheit beeinflußt werden. Diese Parameter können zu Beginn der Applikation fest vorgegeben sein; ferner sind auch Ausführungsformen vorgesehen, bei denen eine nachträgliche oder ständige Steuerung oder Änderung dieser Parameter möglich ist. Bei den genannten Parametern handelt es sich um: Temperatur oder Temperaturbereich (Zieltemperatur); Dauer der einzelnen Wärmepulse; Länge der zwischen den einzelnen Wärmepulsen liegenden Zeitintervalle; Gesamtzahl der Wärmepulse; maximale Gesamtdauer der Behandlung mit Wärmepulsen. Zusätzlich zu den genannten Eigenschaften der Steuereinheit kann diese auch die Steuerung des Stromflusses nach Spannung, Stromstärke und/oder zeitlichem Verlauf ermöglichen. Beispielsweise kann durch die Steuereinheit auch erreicht werden, daß die Form des puls-artige gesteuerten Stromflusses einer Rechteck-, Dreieck-, Sägezahn- oder Sinus-Wellenform entspricht. Der schematische Aufbau einer solchen Steuereinheit ist beispielhaft in Fig. 3 dargestellt.

Die erfindungsgemäßen Vorrichtungen können ferner mit miniaturisierten Wärmefühlern ausgestattet werden, die eine Kontrollmessung der im Heizelement oder auf der Hautoberfläche erreichten Temperatur ermöglichen. Beispielsweise können als Wärmefühler Platin-Temperatursensoren in SMD (Surface Mounted Device)-Bauform und Dünnschichttechnologie mit geringer Wärmekapazität verwendet werden.

Die Ausführung von Steuereinheiten in der beschriebenen Form ist in der elektrotechnischen Fachwelt hinreichend bekannt. Besonderer Wert ist auf eine Leistungsendstufe zu legen, die im ungefährlichen Niedervoltbereich von maximal ca. 24 V die Steuerung von großen Stromflüssen bis zu 100 Ampere und höher zumindest kurzzeitig erlaubt. In dieser Leistungsstufe kommen vorzugsweise Leistungstransistoren, Feldeffekttransistoren oder sogenannte IGBT (Insulated Gate Bipolar Transistor) zum Einsatz, besonders bevorzugt mehrere solcher Bauteile in Parallelschaltung. Ein Beispiel für solche Leistungsstufen, die bereits mit impulsformenden Steuereinheiten integriert sind, liefern sogenannte Fahrtregler, die beispielsweise für Auto-, Flug- und Schiffsmodellbau erhältlich sind.

Bei einem tragbaren System sind vorzugsweise die Stromquelle und die Steuereinheit wegen ihrer Größe räumlich von dem flächigen Heizelement, welches in Verbindung mit dem TTS steht, getrennt. Stromquelle und Steuereinheit können beispielsweise als eigene Einheit am Gürtel, am Handgelenk oder in Taschen der Kleidung getragen werden.
Die Kabelverbindung zwischen der Steuereinheit und dem TTS sollte aus einem Material mit möglichst geringem spezifischem Widerstand ausgeführt werden, so daß große Ströme über kleine Kabelquerschnitte geleitet werden können. Vorzugsweise kommen vergoldete Silberdrahtlitzen oder reiner Golddraht in Betracht.

Als Stromquelle für die Erzeugung der Wärmepulse wird bevorzugt eine galvanische Batterie oder ein Akkumulator, besonders bevorzugt ein Nickel-Cadmium-Akkumulator oder ein Kondensator verwendet. Daneben kommen auch Nickel-Metallhydrid (NiMH)-Akkus und Lithiumionen-Akkus sowie Lithiumbatterien in Betracht.
Wegen der insgesamt sehr geringen Energieaufnahme der erfindungsgemäßen Vorrichtung kann die Stromführungseinrichtung auch transportabel ausgestattet werden. Bei einer typischen Energieverteilung entlädt sich beispielsweise eine Stromquelle mit einem Energieinhalt von 1000 Ws während des Betriebes bzw. der Applikationsdauer 20 mal mit jeweils einer abgegebenen Energiemenge von 50 Ws. Grundsätzlich ist jede elektrische Energiequelle geeignet, die eine ausreichende Kapazität aufweist. Im Falle von Akkumulatoren beträgt diese vorzugsweise 0,1 bis 10 Amperestunden (Ah) bei Spannungen von 1,2 bis 24 V. Für Kondensatoren beträgt sie im selben Spannungsbereich vorzugsweise 0,01 bis 10 F.

Als Heizelemente eignen sich grundsätzlich Materialien bzw. Strukturen, die elektrisch leitfähig sind und eine möglichst geringe Wärmekapazität besitzen. Nur so ist gewährleistet, daß die Hitzepulse in kleinen Quanten, d. h. in Form kurzer und exakter Pulse, möglichst unmittelbar und unverändert auf die Hautoberfläche übertragen werden. Ein Heizelement, welches eine größere Wärmekapazität aufweist, würde die Pulsform der Hitzeerzeugung infolge von Wärmespeicherung verzerren bzw. verfälschen.
Im Falle der Vorrichtungen gemäß Anspruch 1 ist es ferner erforderlich, daß das/die Heizelement(e) wirkstoffdurchlässig sind, damit der Wirkstoff aus dem Wirkstoffreservoir an die Hautoberfläche gelangen kann.

Das Heizelement der erfindungsgemäßen Vorrichtung ist vorzugsweise flächig oder schichtförmig und besonders bevorzugt ein dünner, flächiger Metallfilm; ferner kann auch vorgesehen sein, daß das Heizelement aus mehreren einzelnen solcher Filmflächen aufgebaut ist. Der genannte Metallfilm ist vorzugsweise aus einem Metall hergestellt, das aus der Gruppe ausgewählt ist, die Kupfer, Zinn, Aluminium und andere Weichmetalle, sowie Legierungen aus den genannten Metallen umfaßt. Besonders bevorzugt ist eine Ausführungsform des Metallfilms, wie sie bei der Herstellung von Metallfilmwiderständen mit Widerstandswerten von 0,1 bis 10 Ohm zum Einsatz kommen. Als Modellbeispiele solcher Heizelemente können Widerstände in SMD (Surface Mounted Device) - Bauform betrachtet werden, die als Standardartikel im Elektronikfachhandel erhältlich sind (z. B. Fa. Conrad Elektronik, Hirschau, DE). Derartige Bauteile können in Gruppen auf einer Platinenoberfläche zusammengefügt werden.

Darüber hinaus ist auch vorgesehen, daß das/die Heizelement(e) aus einer elektrisch leitenden Kunststoffolie, oder aus mehreren einzelnen Flächenteilen solcher Folien, besteht. Bevorzugt wird dabei ein mit Kohlestaub hoch angereicherter Polymerfilm eingesetzt.

Falls die Materialien, z. B. Metallfolien, aus denen das Heizelement gefertigt ist, wirkstoffundurchlässig sind, können diese Materialien mit Perforationen, Löchern oder Schlitzen versehen werden, oder gitterartig gestaltet werden oder in mehrere nebeneinander liegende Streifen unterteilt werden. Auf diese Weise wird die erforderliche Durchlässigkeit hergestellt.

Bei einer weiteren Ausführungsform der Erfindung ist vorgesehen, daß als Heizelement eine flächige Wicklung eines dünnen Drahtes, oder mehrere solcher Wicklungen, verwendet wird. Vorzugsweise können dabei Drähte aus Kupfer, Zinn, Aluminium oder anderen Weichmetallen, sowie aus Legierungen aus den genannten Metallen eingesetzt werden.

Im Einzelfall kann es sich auch als vorteilhaft erweisen, wenn die erfindungsgemäßen Vorrichtungen Kombinationen verschiedener Heizelemente enthalten, die aus unterschiedlichen Materialien, wie oben angegeben, hergestellt sind. Auch kann ein einzelnes Heizelement eine Kombination von dafür geeigneten, oben erwähnten Materialien enthalten.

Die Fläche des Heizelements der erfindungsgemäßen Vorrichtungen ist, im Falle der Vorrichtungen des in Anspruch 1 genannten Typs, vorzugsweise möglichst weitgehend identisch mit der Fläche des damit verbundenen TTS. Sie kann bevorzugt 5 bis 100 cm² betragen, besonders bevorzugt 20 bis 50 cm².
Das Heizelement sollte während der Wirkstoffverabreichung möglichst in unmittelbaren Kontakt mit der Hautoberfläche stehen, um die Form und insbesondere die Kürze des Hitzepulses ungedämpft auf die Hautfläche zu übertragen. Dies kann durch dem Fachmann bekannte Maßnahmen erreicht werden, beispielsweise durch eine zusätzliche, auf der hautabgewandten Seite des Heizelements oder des TTS angebrachte haftklebende Fixierschicht oder ein haftklebendes Pflaster. Eine Befestigung der erfindungsgemäßen Vorrichtung auf der Haut kann z. B. auch dadurch ermöglicht werden, daß die Rückschicht des TTS die Fläche des Wirkstoffreservoirs bzw. des flächigen Heizelements überragt, wobei dieser Randbereich auf der hautzugewandten Seite mit einer haftklebenden Beschichtung versehen ist.

Die Wirkstoffdurchlässigkeit des Heizelements wird, falls erforderlich, vorzugsweise dadurch erzielt, daß in dem Heizelement Poren, Löcher, Schlitze oder feine Aussparungen vorgesehen sind, die den Zutritt von Wirkstoffen aus der wirkstoffhaltigen Matrix in das Heizelement und danach in die Haut ermöglichen. Der Wirkstoff kann sich dann durch Querdiffusion in den obersten Hautschichten anreichern und dann während des Hitzepulses die Barriere überwinden.
Der Anteil von freier Fläche (d. h. Poren, Löcher, Schlitze oder Aussparungen) beträgt vorzugsweise 30-70 %, bezogen auf die Gesamtfläche des Heizelements. Die maximale Weite von geschlossenen Flächenabschnitten liegt bevorzugt im Bereich von 100-500 µm, da solche Distanzen von den Wirkstoffmolekülen durch Querdiffusion innerhalb kurzer Zeit überwunden werden können.

Die erfindungsgemäße Vorrichtung nach dem in Anspruch 1 beschriebenen Grundtyp eignet sich grundsätzlich in Verbindung mit den unterschiedlichsten TTS-Bautypen, wie sie eingangs beschrieben wurden. In jedem Fall befindet sich ein wirkstoffdurchlässiges Heizelement oder eine wirkstoffdurchlässige beheizbare Schicht auf der der Haut zugewandten Seite, d. h. der Abgabeseite des TTS.

Die für die Herstellung des Wirkstoffreservoirs bzw. der Wirkstoffmatrix geeigneten Materialien sind dem Fachmann grundsätzlich bekannt, ebenso die Herstellungsverfahren. Als Grundmaterialien für die Wirkstoffmatrix eignen sich vor allem haftklebende Polymere, z. B. Acrylatpolymere oder Acrylatcopolymere, Polyisobutylene, Styrol-Isopren-Blockcopolymere oder Polysilikone, oder auch geeignete Mischungen von Polymeren, oder Heißschmelzkleber.

Die für die Herstellung der Rückschicht des TTS und der ablösbaren Schutzschicht geeigneten Materialien sind dem Fachmann ebenfalls bekannt. Beispielsweise können Folien aus Polyvinylchlorid, Ethylenvinylacetat, Vinylacetat, Polyethylen, Polypropylen oder Cellulosederivaten verwendet werden.
Für die ablösbare Schutzschicht können grundsätzlich dieselben Materialien verwendet werden wie für die Rückschicht, vorausgesetzt, daß diese Schicht einer geeigneten Oberflächenbehandlung, z. B. Fluorosilikonisierung, unterzogen wird, so daß sie von der von ihr bedeckten Haftklebeschicht ablösbar ist und vor Applikation des TTS abgezogen werden kann. Außerdem können als ablösbare Schutzschichten auch andere Materialien verwendet werden, wie z. B. Polytetrafluorethylen-behandeltes Papier, Cellophan, Polyvinylchlorid oder ähnliche.

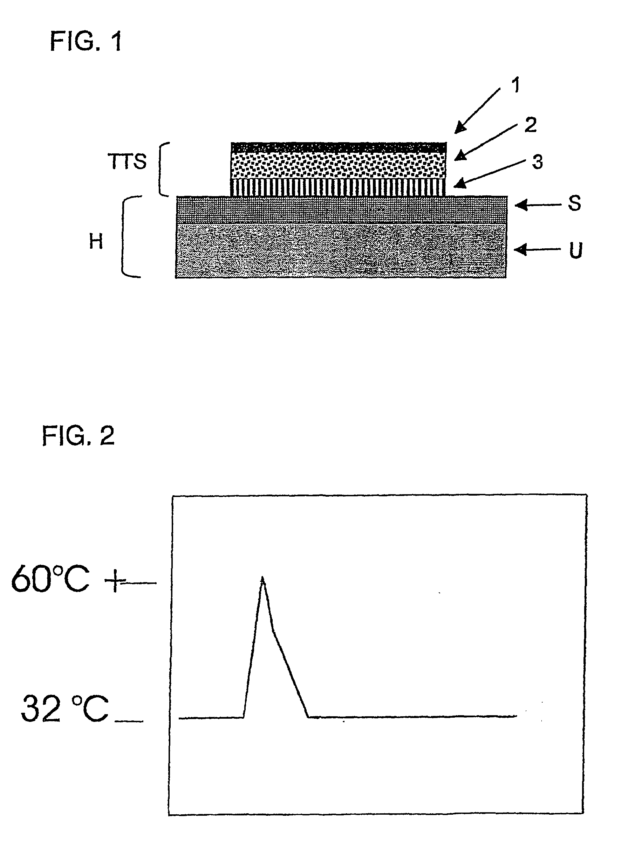

Die Erfindung wird an Hand der Abbildungen (Fig. 1 bis 3) näher erläutert.

Fig. 1 zeigt beispielhaft den Aufbau eines erfindungsgemäßen TTS, und dessen Anordnung auf der Haut (H) während der Applikation (im Schnitt). Dabei bedeuten
(1) die Rückschicht
(2) die wirkstoffhaltige Matrix und
(3) die wirkstoffdurchlässige, mit Hitzepulsen beheizbare Schicht des TTS.
(S) bezeichnet das Stratum corneum der Haut, (U) die Unterhaut.

Fig. 2 zeigt in einer graphischen Darstellung den zeitlichen Temperaturverlauf im Strateum corneum der Haut während der Applikation eines erfindungsgemäßen hitzepuls-gestützten TTS. Die Temperatur im Stratum corneum steigt während der Applikation pulsartig von der normalen Hauttemperatur von 32 °C auf die Zieltemperatur von 60 °C.

Fig. 3 zeigt ein schematisches Blockschaltbild der erfindungsgemäßen Vorrichtungen. Die Anordnung umfaßt eine Stromquelle, die mit der Steuereinheit in Verbindung steht; letztere ist wiederum mit dem Heizelement der Vorrichtung verbunden. Die Steuereinheit weist einen Spannungsregler, eine Strombegrenzung, eine Leistungsendstufe und einen Impulsformer auf. Die gezeigte Anordnung ist lediglich beispielhaft und schränkt die Erfindung in keiner Weise ein.

Die vorliegende Erfindung bezieht sich auch auf Verfahren zur transdermalen Verabreichung von Wirkstoffen, welche darauf beruhen, daß die Applikationsstelle während der Applikationsdauer zur Verbesserung der Wirkstoffpermeation puls-artig aufgeheizt wird, wobei mindestens eine Temperatur von 40 °C erreicht wird. Vorzugsweise wird dies durch Verwendung einer erfindungsgemäßen Vorrichtung nach Anspruch 1 oder 2, wie oben beschrieben, erreicht.

Das Verfahren zur topischen oder transdermalen Verabreichung von Wirkstoffen an die menschliche oder tierische Haut nach Anspruch 14 sieht vor, daß eine Vorrichtung auf die Haut appliziert wird, welche ein transdermales therapeutisches System umfaßt. Aus dem Wirkstoffreservoir dieses TTS wird während der Applikationsdauer der/die Wirkstoffe freigesetzt, wobei die Wirkstoffpermeation durch die Haut mittels puls-artiger Temperaturerhöhungen verbessert wird.

Gemäß einer weiteren Ausführungsform (Anspruch 15) kann das Verfahren zur topischen oder transdermalen Verabreichung von Wirkstoffen an die menschliche oder tierische Haut aber auch auf die Weise durchgeführt werden, daß
- in einem ersten Schritt der/die Wirkstoff(e) in Form einer Wirkstoffzubereitung auf ein Hautareal aufgetragen wird, und
- in einem zweiten Schritt auf die genannte Hautfläche eine Vorrichtung appliziert wird, mittels welcher die Applikationsstelle während der Applikationsdauer zur Verbesserung der Wirkstoffpermeation puls-artig aufgeheizt wird, wobei mindestens eine Temperatur von 40 °C erreicht wird.
Nach diesem Verfahren erfolgt die Verabreichung des Wirkstoffs bzw. der Hitzepulse sequentiell, während in dem Verfahren nach Anspruch 14 der Wirkstoff und die Hitzepulse gleichzeitig verabreicht werden.

Die genannte Wirkstoffzubereitung kann beispielsweise als Lösung, Salbe, Creme oder Gel vorliegen. Es kann sich dabei aber auch um ein wirkstoffhaltiges TTS handeln, welches von dem in Schritt 2 applizierten wirkstofffreien Hitzepuls-Pflaster verschieden ist.

Nach dem Auftragen des Wirkstoffs bzw. der wirkstoffhaltigen Zubereitung (Schritt 1) und gegebenenfalls nach Abwarten einer gewissen Inkubations- oder Einwirkungszeit (10 s bis 24 h, vorzugsweise 1 min bis 1 h) auf der Haut werden, falls erforderlich, die überschüssigen Reste der Wirkstoffzubereitung oberflächlich von der Applikationsstelle entfernt, und sodann das wirkstofffreie Hitzepuls-Pflaster appliziert. Auf diese Weise kann eine homogene und völlig flächenkongruente Applikation von Wirkstoff und Hitzepulsen erreicht werden.
Als Hitzepuls-Pflaster kann vorzugsweise eine Vorrichtung nach Anspruch 2, wahlweise nach einer der in den Unteransprüchen beschriebenen Varianten, verwendet werden.

Durch die vorliegende Erfindung wird eine Steigerung der Hautpermeation von transdermal verabreichten Wirkstoffen ermöglicht, und zwar unabhängig von der Verwendung von permeationsfördernden Zusatzstoffen. Falls eine weitere Steigerung der Hautpermeation erforderlich ist, kann die hitzepuls-gestützte Verabreichung auch mit dem Einsatz von bekannten hautpermeationsfördernden Substanzen kombiniert werden.
Theoretisch ist bei der hitzepuls-gestützten Verabreichung eine Steigerung der Hautpermeation um einen Faktor von 2 bis 100, vorzugsweise von 5 bis 20 zu erwarten.

Die Erfindung kann grundsätzlich für die topische oder transdermale Verabreichung der verschiedenartigsten Wirkstoffe eingesetzt werden. Unter Wirkstoffen werden alle im Bereich der Human- oder Tiermedizin eingesetzten Arzneistoffe verstanden, einschließlich Vitamine, Enzyme und Hormone, sowie Wirkstoffe für kosmetische Behandlungen. Besonders vorteilhaft ist die Verwendung der erfindungsgemäßen Vorrichtung für die Verabreichung von schlecht hautgängigen Substanzen, wie z. B. Peptide, Proteine, Nukleotide, Polynukleotide oder zur Immunisierung geeignete Biomolekülen oder Abschnitte von solchen Biomolekülen. Weiterhin kommen insbesondere solche Wirkstoffe in Betracht, die aufgrund ihrer Molekülmassen von mehr als 500 Da oder aufgrund von Schmelzpunkten oberhalb 200 °C für den Einsatz in passiven TTS wenig geeignet sind.

Die erfindungsgemäße Vorrichtung und das erfindungsgemäße Verfahren eignen sich vorteilhaft für die Verabreichung von Medikamenten zum Zwecke der therapeutischen Behandlung von Mensch oder Tier.

## Patentansprüche

1. Vorrichtung zur hitzepuls-gestützten transdermalen Verabreichung von Wirkstoffen, welche
- ein transdermales therapeutisches System (TTS), das eine Rückschicht, ein wirkstoffhaltiges Reservoir und auf der hautzugewandten Seite ein wirkstoffdurchlässiges elektrisches Heizelement, oder mehrere solche Elemente aufweist,
- und eine mit diesem Heizelement verbundene Steuereinheit und eine Stromquelle umfaßt, wobei die genannte Steuereinheit geeignet ist, ein puls-artiges Aufheizen des genannten Heizelements zu bewirken.

2. Vorrichtung zur Verabreichung von Hitzepulsen an die menschliche oder tierische Haut, welche
- ein haftklebendes medizinisches Pflaster;
- ein oder mehrere elektrische Heizelemente, die mit dem Pflaster verbunden sind und eine Hautkontaktseite aufweisen;
- und eine mit diesem/n Heizelement(en) verbundene Steuereinheit und eine Stromquelle umfaßt, wobei die genannte Steuereinheit geeignet ist, ein puls-artiges Aufheizen des genannten Heizelements zu bewirken.

3. Vorrichtung nach Anspruch 1 oder 2, **dadurch gekennzeichnet, daß** die Aufheizung des Heizelements/der Heizelemente im Temperaturbereich zwischen 40 °C und 200 °C, bevorzugt im Bereich zwischen 50 °C und 120 °C erfolgt, und daß die Dauer der Wärmepulse 0,1 bis 2 Sekunden beträgt, vorzugsweise 0,2 bis 1 s, und wobei die Temperatur und/oder die Dauer der Wärmepulse durch die Steuereinheit vorgegeben oder steuerbar sind.

4. Vorrichtung nach einem der Ansprüche 1 bis 3, **dadurch gekennzeichnet, daß** sie die genannten Wärmepulse wiederholt produziert, wobei die Länge der zwischen den einzelnen Wärmepulsen liegenden Zeitintervalle mindestens 0,01 s, bevorzugt 0,1 s bis 100s, besonders bevorzugt 1 bis 60s beträgt, und wobei die Dauer dieser Zeitintervalle durch die Steuereinheit vorgegeben oder steuerbar sind.

5. Vorrichtung nach einem der vorangehenden Ansprüche, **dadurch gekennzeichnet, daß** als Stromquelle eine oder mehrere galvanische Batterie(n) oder ein oder mehrere Akkumulator(en), vorzugsweise ausgewählt aus der Gruppe der Nikkel-Cadmium-Akkumulatoren, Nickel-Metallhydrid (NiMH)-Akkus, Lithiumionen-Akkus und Lithiumbatterien, oder ein oder mehrere Kondensator(en) verwendet wird bzw. werden, wobei die elektrische Kapazität im Falle von Akkumulatoren vorzugsweise 0,1 bis 10 Ah bei Spannungen von 1,2 bis 24 V und im Falle von Kondensatoren im selben Spannungsbereich vorzugsweise 0,01 bis 10 F beträgt.

6. Vorrichtung nach einem der Ansprüche 1 bis 5, **dadurch gekennzeichnet, daß** das Heizelement flächig oder schichtförmig ist und bevorzugt ein dünner, flächiger Metallfilm ist oder aus mehreren einzelnen solcher Filmflächen aufgebaut ist, wobei der genannte Metallfilm vorzugsweise aus einem Metall hergestellt ist, das aus der Kupfer, Zinn, Aluminium und andere Weichmetalle, sowie Legierungen aus den genannten Metallen umfassenden Gruppe ausgewählt ist.

7. Vorrichtung nach Anspruch 6, **dadurch gekennzeichnet, daß** das Heizelement aus einzelnen dünnen, flächigen Metallfilmen aufgebaut ist, wobei als Metallfilme bevorzugt Metallfilmwiderstände eingesetzt werden.

8. Vorrichtung nach Anspruch 6 oder 7, **dadurch gekennzeichnet, daß** der Metallfilm mit Perforationen, Löchern oder Schlitzen versehen ist, oder daß er gitterartig gestaltet ist oder in mehrere nebeneinander liegende Streifen unterteilt ist.

9. Vorrichtung nach einem der Ansprüche 1 bis 5, **dadurch gekennzeichnet, daß** als Heizelement eine flächige wicklung eines dünnen Drahtes, oder mehrere solcher Wicklungen, verwendet wird, wobei der genannte Draht vorzugsweise aus einem Metall hergestellt ist, das aus der Kupfer, Zinn, Aluminium und andere Weichmetalle, sowie Legierungen aus den genannten Metallen umfassenden Gruppe ausgewählt ist.

10. Vorrichtung nach einem der Ansprüche 1 bis 5, **dadurch gekennzeichnet, daß** das Heizelement im wesentlichen aus einer elektrisch leitenden Kunststoffolie besteht, oder aus mehreren einzelnen Flächenteilen solcher Folien aufgebaut ist, wobei bevorzugt ein mit Kohlestaub hoch angereicherter Polymerfilm eingesetzt wird.

11. Vorrichtung nach einem oder mehreren der vorangehenden Ansprüche, **dadurch gekennzeichnet, daß** die Steuereinheit einen Fahrtregler, eine BEC-Steuerplatine sowie einen Pulsgenerator aufweist.

12. Vorrichtung nach einem oder mehreren der vorangehenden Ansprüche, **dadurch gekennzeichnet, daß** sie während einer Pulsdauer von 0,01 bis 0,5 s, bevorzugt von 0,05 bis 0,1 s, Stromstärken im Bereich von 1 bis 100 A, bevorzugt von 5 bis 20 A, bei einer anliegenden Spannung von 1,2 bis 24 V, bevorzugt von 1,2 bis 7,2 V, erzeugt.

## Claims

1. Device for heat pulse-assisted transdermal administration of active substances, comprising
- a transdermal therapeutic system (TTS), having a backing layer, an active substance-containing reservoir and, on the side facing the skin, an active substance-permeable electric heating element or a plurality of such elements,
- and a control unit, connected to said heating element, and a current source, said control unit being suitable for causing pulse-like heating of said heating element.

2. Device for administration of heat pulses to human or animal skin, comprising
- a pressure-sensitive adhesive medicinal patch;
- one or more electric heating element(s) which are connected with the patch and which have a skin-contact side ;
- and a control unit, connected with said heating element(s), and a current source, said control unit being suitable for effecting pulse-like heating of the said heating element.

3. Device according to claim 1 or 2, **characterized in that** the heating of the heating element/heating elements takes place in the temperature range of between 40 °C to 200 °C, preferably in the range of between 50 °C and 120 °C, and that the duration of the heat pulses is 0.1 to 2 seconds, preferably 0.2 to 1 s, the temperature and/or the duration of the heat pulses being preset by the control unit, or being controllable.

4. Device according to any one of claims 1 to 3, **characterized in that** it produces the said heat pulses repeatedly, the length of the time intervals between the individual heat pulses being at least 0.01 s, preferably 0.1 s to 100 s, with particular preference 1 to 60 s, and the duration of these time intervals being preset or controllable by the control unit.

5. Device according to any one of the preceding claims, **characterized in that** as a source of current, one or more galvanic battery/batteries or one or more accumulator(s), preferably selected from the group of the nickel-cadmium accumulators, nickel-metal hydride (NiMH) accumulators, lithium ion accumulators and lithium batteries, or one or more capacitor(s), is/are used, the electric capacitance in the case of accumulators preferably being 0.1 to 10 Ah at voltages of from 1.2 to 24 V, and in the case of capacitors in the same voltage range, preferably 0.01 to 10 F.

6. Device according to any one of claims 1 to 5, **characterized in that** the heating element is sheet-like or layer-shaped and preferably is a thin, sheet-like metal film or made up of a plurality of such individual film surfaces, the said metal film preferably being made of a metal selected from the group comprising copper, tin, aluminium and other soft metals as well as alloys of the said metals.

7. Device according to claim 6, **characterized in that** the heating element is made up of individual, thin, sheet-like metal films, with metal film resistors preferably being used as the metal films.

8. Device according to claim 6 or 7, **characterized in that** the metal film is provided with perforations, holes or slots, or that it is configured like a lattice, or is divided into a plurality of strips lying next to one another.

9. Device according to any one of claims 1 to 5, **characterized in that** as the heating element a flat-shaped winding of a thin wire, or a plurality of such windings, is used, the said wire preferably being made of a metal selected from the group comprising copper, tin, aluminium and other soft metals, as well as alloys of the said metals.

10. Device according to any one of claims 1 to 5, **characterized in that** the heating element consists substantially of an electrically conductive plastics film, or of a plurality of individual surface parts of such films, with a polymer film high-enriched with carbon powder being used with preference.

11. Device according to one or more of the preceding claims, **characterized in that** the control unit is provided with a speed controller, a BEC control circuit board, as well as a pulse generator.

12. Device according to one or more of the preceding claims, **characterized in that** during a pulse length of 0.01 to 0.5 s, preferably from 0.05 to 0.1 s, it generates current intensities in the range of from 1 to 100 A, preferably from 5 to 20 A, at an applied voltage of 1.2 to 24 V, preferably from 1.2 to 7.2 V.

## Revendications

1. Dispositif pour l'administration transdermique, assistée par des impulsions thermiques, de substances actives qui comprend
- un système thérapeutique transdermique (STT), qui présente une couche arrière, un réservoir contenant la substance active et, sur la face orientée vers la peau, un élément chauffant électrique perméable à la substance active, ou plusieurs de ces éléments, et
- une unité de commande reliée à cet élément chauffant et une source de courant, l'unité de commande étant appropriée pour provoquer un chauffage par impulsions de l'élément chauffant mentionné.

2. Dispositif pour administrer des impulsions de chaleur à la peau humaine ou animale, qui comprend
- un emplâtre médical auto-adhésif ;
- un ou plusieurs éléments chauffants électriques, reliés à l'emplâtre et présentant une face de contact avec la peau ;
- une unité de commande reliée à ce ou ces éléments chauffants et une source de courant, l'unité de commande étant appropriée pour provoquer un chauffage par impulsions de l'élément chauffant mentionné.

3. Dispositif selon la revendication 1 ou 2, **caractérisé en ce que** le chauffage du ou des éléments chauffants est réalisé dans la plage de température entre 40°C et 200°C, de préférence dans la plage entre 50°C et 120°C et la durée des impulsions thermiques est de 0,1 à 2 secondes, de préférence de 0,2 à 1 s, la température et/ou la durée des impulsions thermiques pouvant être prédéfinies ou réglées par l'unité de commande.

4. Dispositif selon l'une quelconque des revendications 1 à 3, **caractérisé en ce qu'**il produit les impulsions thermiques mentionnées de manière répétée, la longueur des intervalles de temps situés entre les différentes impulsions thermiques étant d'au moins 0,01 s, de préférence de 0,1 s à 100 s, de manière particulièrement préférée de 1 à 60 s, et la durée de ces intervalles de temps pouvant être prédéfinie ou réglée par l'unité de commande.

5. Dispositif selon l'une quelconque des revendications précédentes, **caractérisé en ce qu'**on utilise comme source de courant une ou plusieurs piles galvaniques ou un ou plusieurs accumulateurs, de préférence choisis dans le groupe constitué par les accumulateurs au nickel-cadmium, au nickel-hydrure de métal (NiMH), aux ions de lithium et les piles au lithium, ou un ou plusieurs condensateurs, la capacité électrique dans le cas des accumulateurs étant de préférence de 0,1 à 10 Ah à des tensions de 1,2 à 24 V et, dans le cas des condensateurs dans la même plage de tension, de préférence de 0,01 à 10 F.

6. Dispositif selon l'une quelconque des revendications 1 à 5, **caractérisé en ce que** l'élément chauffant est plat ou en forme de couches et est de préférence un film métallique mince, plat ou constitué par plusieurs de ces surfaces de film, le film métallique mentionné étant de préférence réalisé à partir d'un métal qui est choisi dans le groupe constitué par le cuivre, l'étain, l'aluminium et d'autres métaux souples ainsi que les alliages des métaux mentionnés.

7. Dispositif selon la revendication 6, **caractérisé en ce que** l'élément chauffant est constitué par plusieurs films métalliques minces, plats, en utilisant comme film métallique de préférence des résistances à film métallique.

8. Dispositif selon la revendication 6 ou 7, **caractérisé en ce que** le film métallique est pourvu de perforations, de trous ou de fentes ou est réalisé en forme de grille ou est divisé en plusieurs bandes adjacentes.

9. Dispositif selon l'une quelconque des revendications 1 à 5, **caractérisé en ce qu'**on utilise comme élément chauffant une bobine plate d'un fil mince ou plusieurs de ces bobines, le fil mentionné étant de préférence réalisé à partir d'un métal choisi dans le groupe constitué par le cuivre, l'étain, l'aluminium et d'autres métaux souples ainsi que les alliages des métaux mentionnés.

10. Dispositif selon l'une quelconque des revendications 1 à 5, **caractérisé en ce que** l'élément chauffant est essentiellement constitué par une feuille synthétique électriquement conductrice ou plusieurs parties planes différentes de ces feuilles, en utilisant de préférence un film polymère hautement enrichi en poussière de carbone.

11. Dispositif selon l'une ou plusieurs des revendications précédentes, **caractérisé en ce que** l'unité de commande présente un régulateur de vitesse, une carte électronique de commande à rallonge pour communication numérique et signaux analogiques ainsi qu'un générateur d'impulsions.

12. Dispositif selon l'une ou plusieurs des revendications précédentes, **caractérisé en ce qu'**il produit pendant une durée d'impulsion de 0,01 à 0,5 s, de préférence de 0,05 à 0,1 s, des intensités de courant dans la plage de 1 à 100 A, de préférence de 5 à 20 A, à une tension appliquée de 1,2 à 24 V, de préférence de 1,2 à 7,2 V.
